# EUROPEAN PATENT SPECIFICATION

(11) **EP 0 936 921 B1**
(45) Date of publication and mention of the grant of the patent: **19.03.2003**
(21) Application number: 97930276.7
(22) Date of filing: 11.07.1997
(51) Int. Cl.: A61K 39/155

(54) **TWO-STEP IMMUNIZATION PROCEDURE AGAINST THE PYRAMYXOVIRIDAE FAMILY OF VIRUSES USING ATTENUATED VIRAL STRAINS AND SUBUNIT PROTEIN PREPARATION**
IMMUNISIERUNGSVERFAHREN IN ZWEI SCHRITTEN GEGEN PYRAMYXOVIRIDAE-VIREN UNTER VERWENDUNG ABGESCHWÄCHTER VIRALER STÄMME UND PREPARATION VON PROTEINUNTEREINHEITEN
PROCEDURE D'IMMUNISATION EN DEUX ETAPES CONTRE LA FAMILLE PYRAMYXOVIRIDAE DE VIRUS A L'AIDE DE SOUCHES VIRALES ATTENUEES ET D'UNE PREPARATION PROTEIQUE DE SOUS-UNITES

(30) Priority: 12.07.1996 US 679206
(43) Date of publication of application: 25.08.1999
(73) Proprietor: Aventis Pasteur Limited, Toronto, Ontario M2R 3T4 (CA); THE REGENTS OF THE UNIVERSITY OF MICHIGAN, Ann Arbor Mi 48109-8202 (US)
(72) Inventor: KLEIN, Michel, H., Willowdale, Ontario M2P 1B9 (CA); CATES, George, A., Richmond Hill, Ontario L4C 3T5 (CA); EWASYSHYN, Mary, E., Willowdale, Ontario M2R 3N7 (CA); HERLOCHER, M., Louise, Ann Arbor, MI 48103 (US); MAASSAB, H., F., Ann Arbor, MI 48104 (US)
(74) Representative: Williams, Richard Andrew Norman
(86) International application number: CA9700499
(87) International publication number: WO98002180

(56) References cited:
- WO-A-87/04185
- WO-A-93/21310
- WO-A-94/27636
- EWASYSHYN M.E. AND KLEIN M.H.: "Prospects for Immunization Against Respiratory Syncytial Virus" VACCINE RESEARCH, vol. 2, 1993, pages 263-272, XP002044888

## Description

### FIELD OF INVENTION

The present invention relates to the field of immunology and, in particular, to a vaccination procedure for protection of a host against disease caused by infection with a virus belonging to the paramyxoviridae family of viruses, particularly the paramyxovirus, particularly respiratory syncytial virus (RSV).

### BACKGROUND TO THE INVENTION

Human parainfluenza virus type 1, 2, 3 and human respiratory syncytial virus (RSV) have been identified as the major viral pathogens responsible for severe respiratory tract infections in infants and young children (ref. 1 to 3 - Throughout this specification, various references are referred to in parenthesis to more fully describe the state of the art to which this invention pertains. Full bibliographic information for each citation is found at the end of the specification, immediately following the claims. RSV has also been reported to cause significant morbidity in immunocompromised individuals and the elderly. Globally 65 million infections occur every year resulting in 160,000 deaths (ref. 4). In the USA alone, 100,000 children are hospitalized annually with severe cases of pneumonia and bronchiolitis resulting from an RSV infection (refs. 5, 6). Inpatient and ambulatory care for children with RSV infections has been estimated to cost in excess of $340 million each year in the USA (ref. 7). Severe lower respiratory tract disease due to RSV infection predominantly occurs in infants two to six months of age (ref. 8). The World Health Organization (WHO) and the National Institute of Allergy and Infectious Disease (NIAID) vaccine advisory committees have ranked RSV second only to HIV for vaccine development while the preparation of an efficacious PIV-3 vaccine is ranked in the top ten vaccines considered a priority for vaccine development. Both the annual morbidity and mortality figures as well as the staggering health care costs for managing paramyxoviridae infections, including RSV and PIV, have provided the incentive for aggressively pursuing the development of efficacious vaccines.

RSV is a member of the Paramyxoviridae family of pneumovirus genus (ref. 2). The two major protective antigens of RSV are the envelope fusion (F) and attachment (G) glycoproteins (ref. 9). In addition, to the antibody response generated by the F and G glycoproteins, human cytotoxic T-cells have been shown to recognize the RSV fusion protein (F), matrix (M) protein, nucleoprotein (N), small hydrophobic protein (SH) and nonstructural protein (lb) (ref. 10). For PIV-3, the protective immunogens are the hemagglutinin-neuramidase (HN) protein and the fusion (F) protein.

Previous attempts to produce a safe and effective RSV vaccine were unsuccessful. Production of live attenuated RSV vaccines has had limited success. Several different strategies have been used in an attempt to produce avirulent, immunoprotective and genetically-stable live attenuated mutants of RSV. These approaches have included treating RSV with various chemical mutagens, passaging the virus multiple times at either a specific temperature or at progressively lower temperatures and a combination of the aforementioned approaches. Such approaches have resulted in the product of attenuated viruses which exhibit the cold-adapted (*ca*) and/or temperature sensitive (*ts*) phenotype. The mutants prepared to date have all been either over-attenuated, virulent or genetically unstable. A formalin-inactivated (FI) RSV vaccine developed in the 1960's failed to provide adequate protection in clinical trials (refs. 8, 11, 12). In fact, immunization of seronegative infants with the FI-RSV vaccine resulted in the exacerbation of RSV disease (immunopotentiation) in some vaccinees following exposure to wild type virus. Identification of the major immunoprotective antigens of RSV has provided the scientific rationale for pursuing a subunit approach to RSV vaccine development. However, efficacy of the RSV subunit vaccines tested to date have been inconsistent (ref. 12). There are also conflicting reports in the literature on the ability of an alum-adjuvanted RSV vaccine containing the F protein purified from virus-infected cells by immunoaffinity chromatography (PFP-1) to cause enhanced pulmonary pathology (immunopotentiation) following live virus challenge (ref. 13, 14). There is a definite requirement for the development of a safe and efficacious RSV vaccine.

One of the main obstacles in developing a safe and effective RSV vaccine has been to produce a vaccine formulation that can elicit a protective immune response without causing exacerbated disease. Elucidation of the mechanism(s) involved in the potentiation of RSV disease is important for the design of safe RSV vaccines, especially for the seronegative population. Recent experimental evidence suggests that an imbalance in cell-mediated responses may contribute to immunopotentiation (ref. 15). Enhanced histopathology observed in mice that were immunized with the FI-RSV and challenged with virus could be abrogated by depletion of CD4₊ T-cells or both interleukin-4 (IL-4) and IL-10 (ref. 16). Experimental results indicated that induction of a Th-2 type response may play a role in disease potentiation. BALB/c mice given live virus intranasally or intramuscularly elicited a Th-1 type response, whereas FI-RSV induced a Th-2 type of response. These results were recently substantiated by the finding that BALB/c mice that were immunized with the FI-RSV vaccine had a marked increase in the expression of mRNA (from cells in the bronchoalveolar lavage fluid) for the Th-2 cytokines IL-5 and IL-13 (ref. 17).

Studies on the development of live viral vaccines and glycoprotein subunit vaccines against parainfluenza virus infection are being pursued. Clinical trial results with a formalin-inactivated PIV types 1,2,3 vaccine demonstrated that this vaccine was not efficacious (refs. 18, 19, 20). Further development of chemically-inactivated vaccines was discontinued after clinical trials with a formalin-inactivated RSV vaccine demonstrated that not only was the vaccine not effective in preventing RSV infection but many of the vaccinees who later became infected with RSV suffered a more serious disease. Most of parainfluenza vaccine research has focussed on candidate PIV-3 vaccines (ref. 21) with significantly less work being reported for PIV-1 and PIV-2. Recent approaches to PIV-3 vaccines have included the use of the closely related bovine parainfluenza virus type 3 and the generation of attenuated viruses by cold-adaptation of the virus (refs. 22, 23, 24, 25).

Another approach to parainfluenza virus type 3 vaccine development is a subunit approach focusing on the surface glycoproteins hemagglutinin-neuraminidase (HN) and the fusion (F) protein (refs. 26, 27, 28). The HN antigen, a typical type II glycoprotein, exhibits both haemagglutination and neuraminidase activities and is responsible for the attachment of the virus to sialic acid containing host cell receptors. The type I F glycoprotein mediates fusion of the viral envelope with the cell membrane as well as cell to cell spread of the virus. It has recently been demonstrated that both the HN and F glycoproteins are required for membrane fusion. The F glycoprotein is synthesized as an inactive precursor (F) which is proteolytically cleaved into disulfide-linked F2 and F1 moieties. While the HN and F proteins of PIV-1, 2 and 3 are structurally similar, they are antigenically distinct. Neutralizing antibodies against the HN and F proteins of one of PIV type are not cross-protective. Thus, an effective PIV subunit vaccine must contain the HN and F glycoproteins from the three different types of parainfluenza viruses. Antibody to either glycoprotein is neutralizing *in vitro.* A direct correlation has been observed between the level of neutralizing antibody titres and resistance to PIV-3 infections in infants. Native subunit vaccines for parainfluenza virus type 3 have investigated the protectiveness of the two surface glycoproteins. Typically, the glycoproteins are extracted from virus using non-ionic detergents and further purified using lectin affinity or immunoaffinity chromatographic methods. However, neither of these techniques may be entirely suitable for large scale production of vaccines under all circumstances. In small animal protection models (hamsters and cotton rats), immunization with the glycoproteins was demonstrated to prevent infection with live PIV-3 (refs. 29, 30, 31, 32, 33). The HN and F glycoproteins of PIV-3 have also been produced using recombinant DNA technology. HN and F glycoproteins have been produced in insect cells using the baculovirus expression system and by use of vaccinia virus and adenovirus recombinants (refs. 34, 35, 36, 37, 38). In the baculovirus expression system, both full-length and truncated forms of the PIV-3 glycoproteins as well as a chimeric F-HN fusion protein have been expressed. The recombinant proteins have been demonstrated to be protective in small animal models (see WO91/00104. and EP-A-0 480 949 assigned to the assignee hereof).

### SUMMARY OF THE INVENTION

The present invention provide a novel composition for a novel immunization strategy to provide protection against disease caused by infection with, respiratory syncytial virus (RSV). The immunization strategy described herein leads to a stronger protective immune response than other strategies and this strategy comprises:
initially administering to the host an immunoeffective amount of an attenuated strain of RSV and
subsequently administering to the host an immunoeffective amount of at least one purified RSV protein or immunogenic fragment thereof of the same virus as used in the initial administration, to achieve a virus specific protective immune response in the host.

The immune response which is achieved in the host by the method of the invention preferably includes the production of virus specific neutralizing antibodies and the virus specific cytotoxic T-cell responses. The immunization strategy described herein may lead to the induction of a more balanced anti-F IgGl/IgG2a response, demonstrating the induction of both a Th-1 and Th-2 type response than previously attained.

The compositions of the invention are particularly effective to provide protection against respiratory tract diseases caused by respiratory syncytial virus (RSV). The attenuated strain of RSV may be a temperature sensitive cold-adapted, or host-range restricted or genetically modified mutant of RSV.

In an embodiment, the RSV protein or immunogenic fragment thereof employed in the second or booster administration and selected from the group consisting of the F, G and M proteins of RSV or immunogenic fragments thereof and may comprise a mixture of two or three of these RSV proteins or the immunogenic fragments thereof.

The at least one purified paramyxovirus protein or immunogenic fragment thereof, preferably mixtures of the F, G and/or M proteins of RSV or immunogenic fragments thereof, may be administered with an adjuvant, such as alum.

### BRIEF DESCRIPTION OF DRAWINGS

Figure 1, containing panels a and b, shows SDS-PAGE analysis of a purified RSV subunit A preparation using polyacrylamide gels stained with silver, under both reduced (panel (a)) and non-reduced (panel (b)) conditions;
Figure 2, containing panels a, b, c and d, shows Western blot analysis of a purified RSV subunit preparation under reduced conditions;
Figure 3, containing panels a, b, c and d, shows Western blot analysis of a purified RSV subunit preparation under non-reduced conditions;
Figure 4, containing panels A, B and C, shows anti-RSV F titres in the sera of mice immunized with different prime/boost protocols, as detailed in Table 2 below;
Figure 5 shows plaque reduction titres in the sera of mice immunized and different prime/boost protocols, as detailed in Table 2 below; and
Figure 6 shows the cytotoxic T lymphocyte responses in mice immunized with different prime/boost protocols, as detailed in Table 2 below.

### GENERAL DESCRIPTION OF THE INVENTION

The present invention relates to compositions for use in methods of immunization comprising adminstration of an attenuated strain of RSV and subsequent administration of at least one protein or fragment thereof of RSV. The RSV may itself be overattenuated and may be unable to confer protection.

In one aspect, the at least one protein may comprise a mixture of RSV proteins such as G glycoprotein, F glycoprotein, M protein, as well as heterodimers and oligomeric forms of the F and G proteins. Thus, the subsequent administration may be of a subunit preparation isolated from RSV viral concentrates.

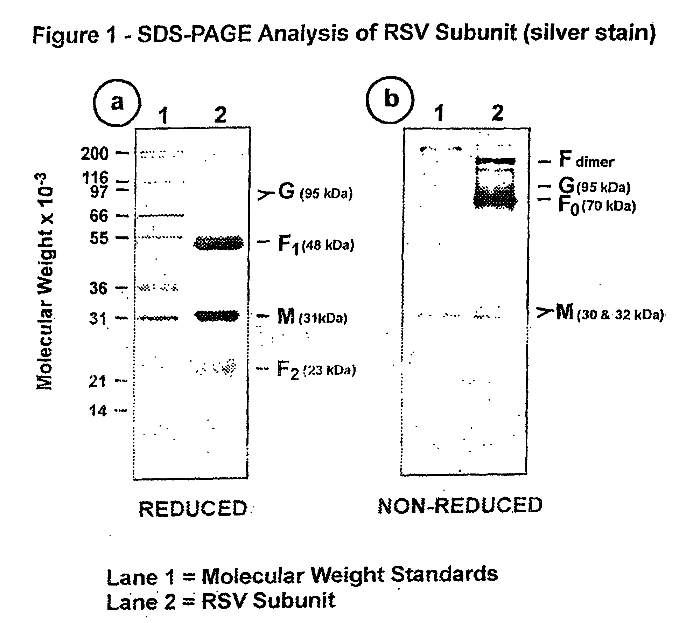

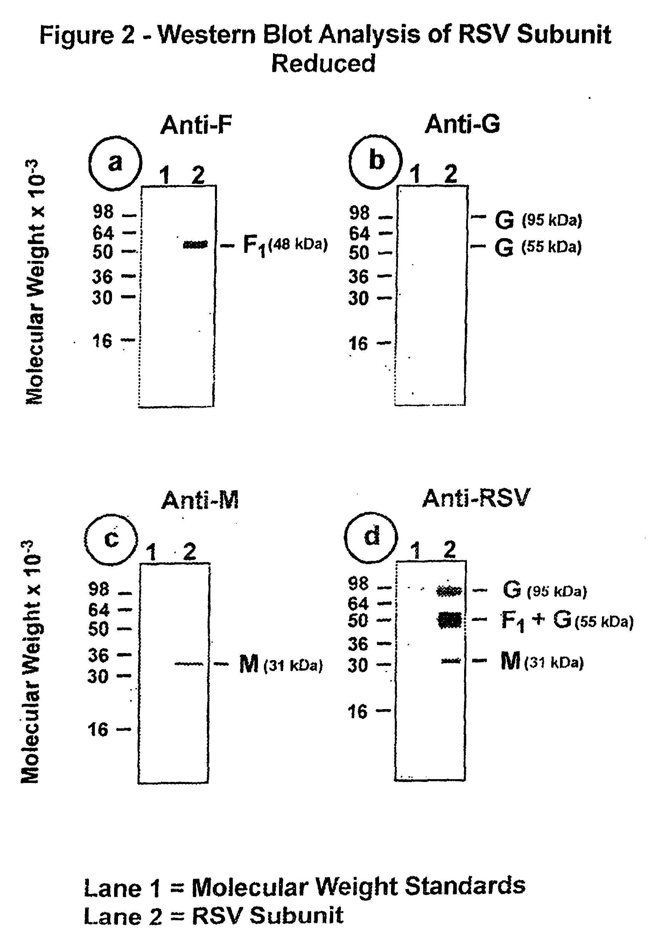

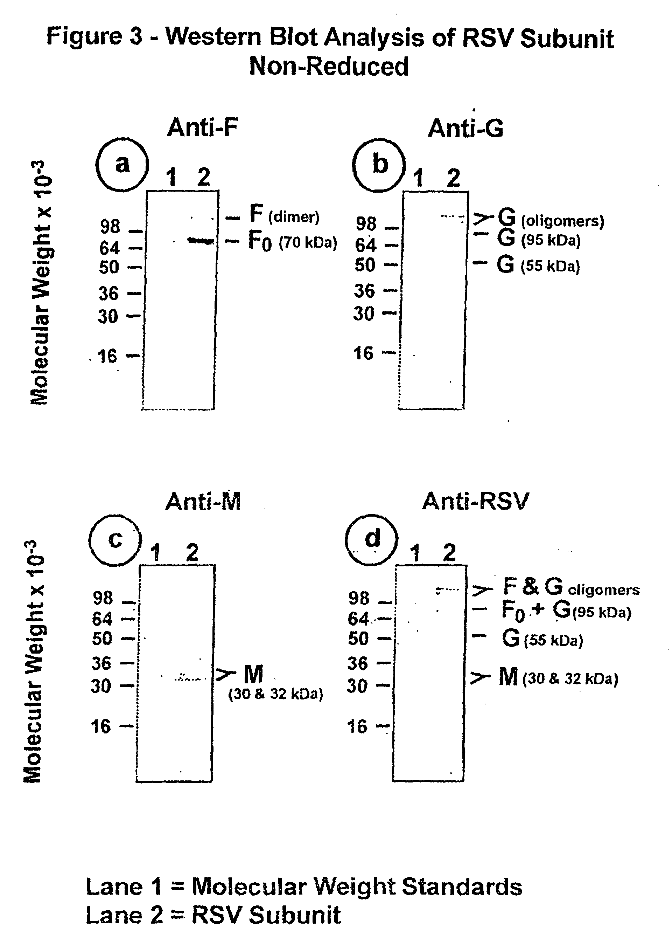

Referring to Figure 1 to 3, there is shown an analysis of the composition of a RSV subunit preparation by SDS-PAGE analysis (Figure 1) and immunoblot (Figures 2 and 3), prepared as described below. A typical composition of the RSV subunit preparation is:

| | | |
|---|---|---|
| G | glycoprotein (95 kDa form) | 10% |
| F₁ | glycoprotein (48 kDa) | 30% |
| M | protein (31 kDa) | 23% |
| F₂ | glycoprotein (23 kDa) | 19% |

when analyzed by SDS-PAGE under reduced conditions.

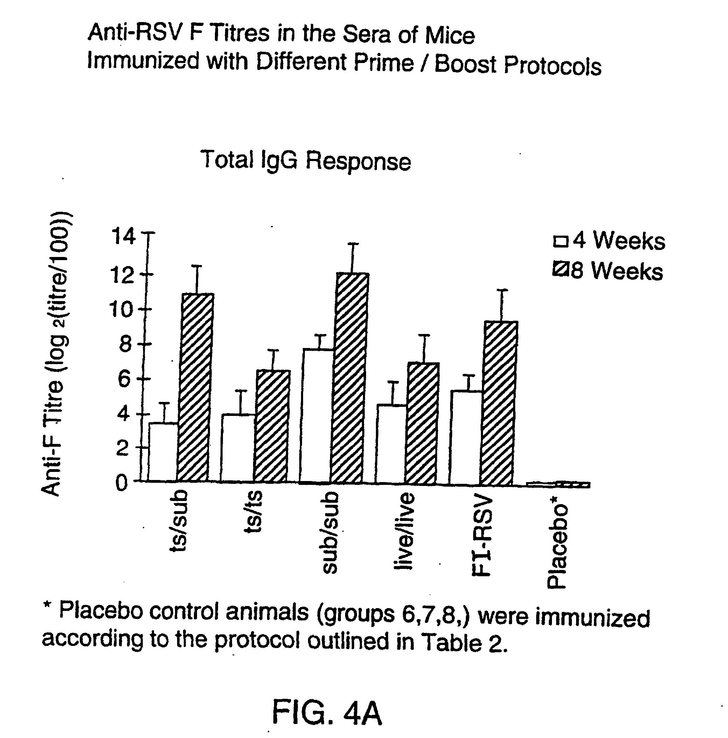

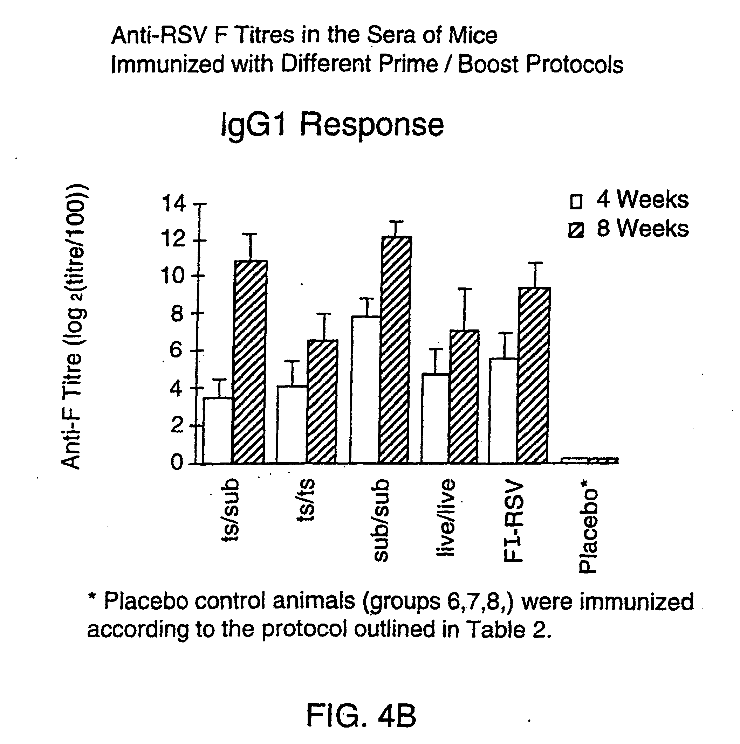

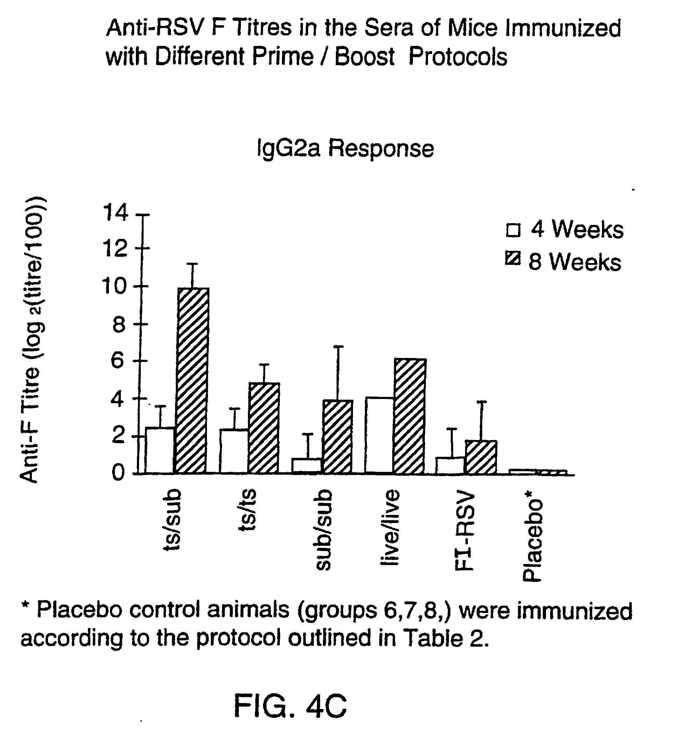

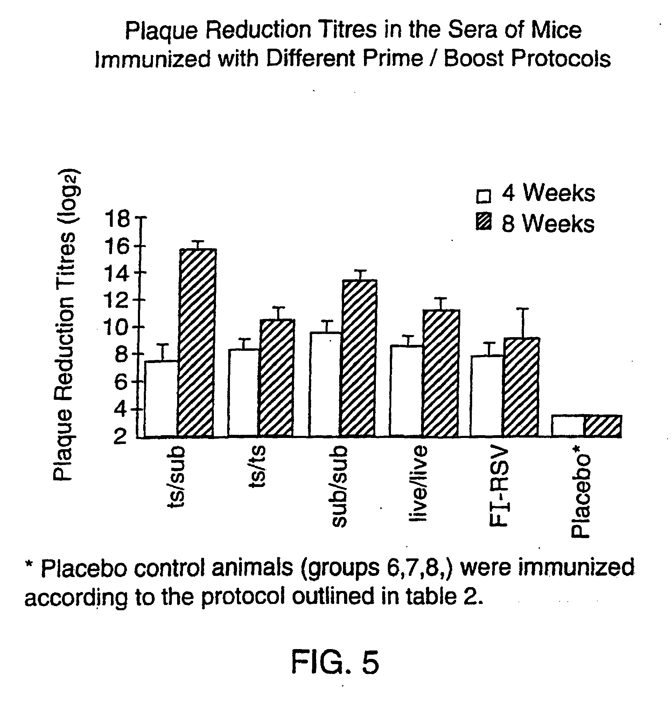

Mice were immunized by priming with an attenuated temperature sensitive RSV strain and boosted with the mixture of RSV proteins (RSV subunit preparation) as summarized in Table 2. Sera were examined four weeks after boosting for anti-F total IgG antibodies, IgG1 and IgG2a antibodies. The results are shown in Figure 4 and show that the intranasal immunization with the attenuated RSV followed by boosting with the subunit RSV protein preparation produces a substantial anti-F antibody response. In particular, a balanced anti-RSV F IgG1/IgG2a response demonstrating the induction of both Th-1 and Th-2 type responses is achieved. The generation of IgG2A antibodies in the murine model is indicative of a Th1-type immune response. The level of virus-neutralizing antibodies was determined by plaque reduction assays (Figure 5).

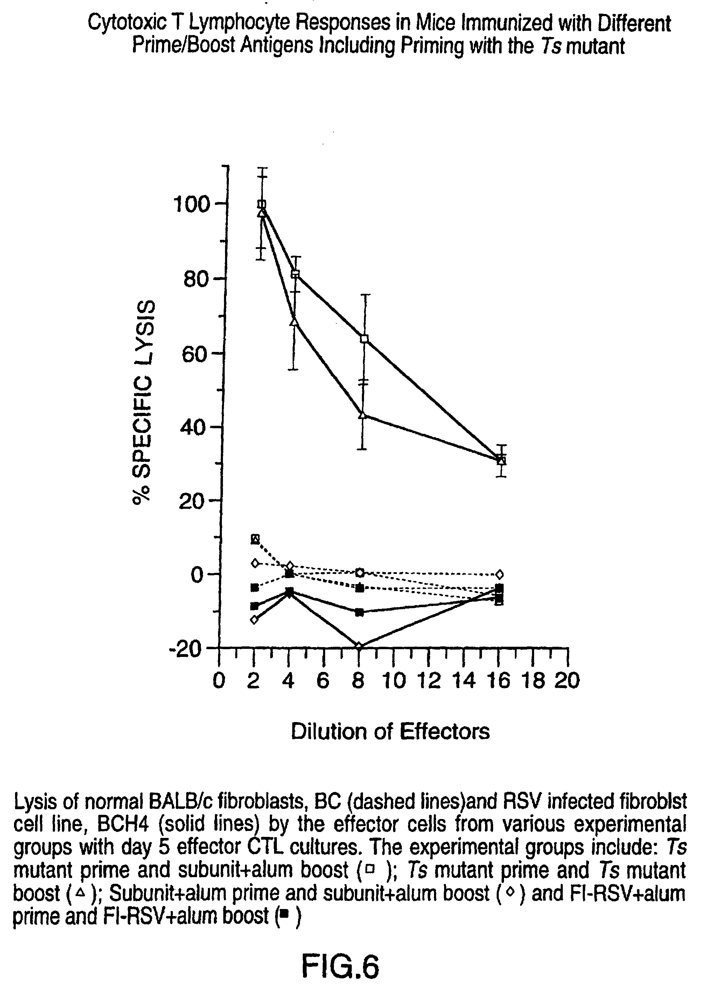

The highest RSV-specific neutralizing antibody titres were observed in animals that were primed with the attenuated temperature sensitive (*ts*) RSV mutant and boosted with the RSV subunit preparation. The generation of RSV-specific cytotoxic T cells (CTL) following immunization was determined and the results shown in Figure 6. No CTL activity was observed in animals that were primed and boosted with either alum-adjuvanted RSV subunit vaccine or formalin-inactivated RSV.

In contrast, priming animals with the attenuated ts RSV mutant and boosting them with either attenuated RSV or an RSV subunit preparation induced significant levels of CTL activity.

The invention extends to compositions comprising an attenuated RSV to prime a host and the subsequent use of at least one RSV protein to boost the host to immunize the host against disease caused by the RSV.

The vaccines are administered in a manner compatible with the dosage formulation, and in such amount as will be therapeutically effective, immunogenic and protective. The quantity to be administered depends on the subject to be treated, including, for example, the capacity of the immune system of the individual to synthesize antibodies, and, if needed, to produce a cell-mediated immune response. Precise amounts of active ingredient required to be administered depend on the judgment of the practitioner. However, suitable dosage ranges are readily determinable by one skilled in the art and may be of the order of micrograms to milligrams of the proteins or fragments thereof and 10⁵ - 10⁸ pfu of the attenuated virus. The dosage may also depend on the route of administration and will vary according to the size of the host.

Immunogenicity can be significantly improved if the antigens are co-administered with adjuvants. Adjuvants enhance the immunogenicity of an antigen but are not necessarily immunogenic themselves. Adjuvants may act by retaining the antigen locally near the site of administration to produce a depot effect facilitating a slow, sustained release of antigen to cells of the immune system. Adjuvants can also attract cells of the immune system to an antigen depot and stimulate such cells to elicit immune responses.

Immunostimulatory agents or adjuvants have been used for many years to improve the host immune responses to, for example, vaccines. Intrinsic adjuvants, such as lipopolysaccharides, normally are the components of the killed or attenuated bacteria used as vaccines. Extrinsic adjuvants are immunomodulators which are typically non-covalently linked to antigens and are formulated to enhance the host immune responses. Thus, adjuvants have been identified that enhance the immune response to antigens delivered parenterally. Some of these adjuvants are toxic, however, and can cause undesirable side-effects, making them unsuitable for use in humans and many animals. Indeed, only aluminum hydroxide and aluminum phosphate (collectively commonly referred to as alum) are routinely used as adjuvants in human and veterinary vaccines. The efficacy of alum in increasing antibody responses to diphtheria and tetanus toxoids is well established and a HBsAg vaccine has been adjuvanted with alum. While the usefulness of alum is well established for some applications, it has limitations. For example, alum is ineffective for influenza vaccination and usually does not elicit a cell-mediated immune response. The antibodies elicited by alum-adjuvanted antigens are mainly of the IgG1 isotype in the mouse, which may not be optimal for protection by some vaccinal agents.

A wide range of extrinsic adjuvants can provoke potent immune responses to antigens. These include aluminum phosphate, aluminum hydroxide, QS21, Quil A, derivatives and components thereof, calcium phosphate, calcium hydroxide, zinc hydroxide, a glycolipid analog, an octodecyl ester of an amino acid, a muramyl dipeptide, polyphosphazene, a lipoprotein, ISCOM matrix, DC-Chol, DDA, and other adjuvants and bacterial toxins, components and derivatives thereof as, for example, described in WO 95/34323 assigned to the assignee hereof. Under particular circumstances adjuvants that induce a Thl response are desirable.

The at least one purified paramyxovirus protein or immunogenic fragment thereof used in the booster administration may be provided in any convenient manner. For example, the protein, proteins or immunogenic fragments may be extracted from cellular material, such as by detergent extraction, and may be purified by affinity chromatography, as described, for example, in WO 91/00104, assigned to the assignee hereof Alternatively, an immunoaffinity purification procedure may be used.

Alternatively, the protein, proteins or immunogenic fragments thereof may be prepared recombinantly by expression of the protein or fragment thereof from a suitable vector with subsequent purification of the expressed material. Suitable vectors and expression systems are described, for example, in WO 87/04185.

In general, plasmid vectors containing replicon and control sequences which are derived from species compatible with the host cell may be used for the expression of the relevant genes in expression systems. The vector ordinarily carries a replication site, as well as marking sequences which are capable of providing phenotypic selection in transformed cells. Details concerning the nucleotide sequences of promoters are known, enabling a skilled worker to ligate them functionally with the relevant genes. The particular promoter used will generally be a matter of choice depending upon the desired results. Hosts that are appropriate for expression of the relevant genes and immunogenic fragments thereof include bacteria, eukaryotic cells, fungi, yeast, CHO cells or the baculovirus expression system may be used.

### Biological Deposits

A temperature sensitive mutant strain of RSV which is described and referred to herein has been deposited with the American Type Culture Collection (ATCC) located at 12301 Parklawn Drive, Rockville, Maryland 20852, USA pursuant to the Budapest Treaty and prior to the filing of this application. Samples of the deposited strain will become available to the public and all restrictions imposed on access to the deposits will be removed upon grant of a patent on this application. The invention described and claimed herein is not to be limited in scope by the mutant RSV strains deposited, since the deposited embodiment is intended only as an illustration of the invention. Any equivalent or similar recombinant viruses are within the scope of the invention.

### Deposit Summary

| **Recombinant Virus** | **ATCC Designation** | **Date Deposited** |
|---|---|---|
| Line 19 MRC-56-35 | VR-2513 | 20 September 1995 |

### EXAMPLES

The above disclosure generally describes the present invention. A more complete understanding can be obtained by reference to the following specific Examples. These Examples are described solely for purposes of illustration and are not intended to limit the scope of the invention. Changes in form and substitution of equivalents are contemplated as circumstances may suggest or render expedient. Although specific terms have been employed herein, such terms are intended in a descriptive sense and not for purposes of limitations.

### Example 1:

This Example describes the preparation of the live attenuated *ts* mutant

MRC-5 cells (grown in 16 x 125 mm tubes) were obtained from BioWhittaker Laboratories. (Walkersville, MD). Medium was removed from 10-day old cultures and 1.2 mL EMEM + 5% fetal bovine serum (FBS) was added to each tube. A 0.3 mL aliquot of a clinical isolate of RSV subtype A (designated line 19 from clinical case 19413, I.D. 12868) that was first isolated in WI-38 cells and passaged 7 times at 25°C in WI-38 cells was added to each of 4 tubes of MRC-5 cells. Virus-infected cells were incubated at 35°C and fed with 0.2 mL EMEM + 5% FBS as required. When the cytopathic effect (CPE) was extensive, (usually 4 to 5 days post-infection) tubes of virus-infected cells were frozen at -70°C. Virus was harvested and used to infect MRC-5 cells. The virus was passaged 56 times in MRC-5 cells at 35°C using the aforementioned procedure. As demonstrated in Table 1 this RSV mutant (designated Line 19 MRC-56-35) exhibited the *temperature sensitive* (*ts*) phenotype since the infectivity titre at 39°C was approximately 3 logs lower than at 33°C. This is in contrast to the wild type virus (designated WRSV MRC) which had the same infectivity titre at 33°C and 39°C. This *ts* attenuated RSV strain was deposited with the ATCC and given the accession No. VR-2513.

### Example 2:

This Example illustrates the process of purifying RSV proteins.

African Green monkey kidney cells (VERO) at a concentration of 10⁵ cells/mL were added to 60L of CMRL 1969 medium, pH 7.2 in a 150L bioreactor containing 360 g of Cytodex-1 (RTM) microcarrier beads and stirred for 2 hours. An additional 60L of CMRL 1969 was added to give a total volume of 120L. Fetal bovine serum was added to achieve a final concentration of 3.5%. Glucose was added to a final concentration of 3 g/L and L-glutamine was added to a final concentration of 0.6 g/L. Dissolved oxygen (40%), pH (7.2), agitation (36 rpm), and temperature (37°C) were controlled. Cell growth, glucose, lactate, and glutamine levels were monitored. At day 4, the culture medium was drained from the fermenter and 100L of E199 media (without fetal bovine serum) was added and stirred for 10 minutes. The fermenter was drained and filled again with 120 L of E199. The RSV inoculum was added at a multiplicity of infection (M.O.I.) of 0.001 and the culture was then maintained for 3 days before one-third to one-half of the medium was drained and replaced with fresh medium. On day 6 post-infection, the stirring was stopped and the beads allowed to settle. The viral culture fluid was drained and filtered through a 20 µm filter followed by a 3 µm filter prior to further processing. The clarified viral harvest was concentrated 75- to 150-fold using tangential flow ultrafiltration with 300 NMWL membranes and diafiltered with phosphate buffered saline containing 10% glycerol. The viral concentrate was stored frozen at -70°C prior to further purification. A solution of 50% polyethylene glycol-8000 was added to an aliquot of virus concentrate to give a final concentration of 6%. After stirring at room temperature for one hour, the mixture was centrifuged at 15,000 RPM for 30 min. in a Sorvall SS-34 rotor at 4°C. In some instances the viral pellet was suspended in 1 mM sodium phosphate, pH 6.8, 2 M urea, 0.15 M NaCl, stirred for 1 hour at room temperature, and then recentrifuged at 15,000 RPM for 30 min. in a Sorvall SS-34 rotor at 4°C. The viral pellet was then suspended in 1mM sodium phosphate, pH 6.8, 50 mM NaCl, 1% Triton X-100 (RTM) and stirred for 30 min. at room temperature. The insoluble virus core was removed by centrifugation at 15,000 RPM for 30 min. in a Sorval SS-34 rotor at 4°C. The soluble protein supernatant was applied to a column of ceramic hydroxyapatite (type II, Bio-Rad Laboratories) and the column was then washed with five column volumes of 1 mM sodium phosphate, pH 6.8, 50 mM NaCl, 0.02% Triton X-100. The RSV proteins were obtained by eluting the column with 10 column volumes of 1 mM sodium phosphate, pH 6.8, 400 mM NaCl, 0.02% Triton X-100.

The RSV proteins were analyzed by SDS-PAGE using 12.5% acrylamide gels and by immunoblotting. Samples were electrophoresed in the presence or absence of the reducing agent 2-mercaptoethanol. Gels were stained with silver stain to detect the viral proteins (Figures 1a and 1b). Immunoblots of replicate gels were prepared and probed with a mouse monoclonal antibody (mAb 5353C75) to F glycoprotein (Figures 2a and 3a), or a mouse monoclonal antibody (mAb 131-2G), to G glycoprotein (Figures 2b and 3b) or guinea pig anti-serum (gp178) against an RSV M peptide (peptide sequence: LKSKNMLTTVKDLTMKTLNPTHDIIALCEFEN - SEQ ID No: 1) (Figures 2c and 3c), or goat anti-serum (Virostat #0605) against whole RSV (Figures 2d and 3d). Densitometric analysis of the silver-stained gel of the RSV subunit preparation electrophoresed under reducing conditions indicated a compositional distribution as follows:
G glycoprotein (95 kDa form) = 10 %
F₁ glycoprotein (48 kDa) = 30 %
M protein (31 kDa) = 23 %
F₂ glycoprotein (23 kDa) = 19 %
The F glycoprotein migrates under non-reducing conditions as a heterodimer of approximately 70 kDa (F₀) as well as higher oligomeric forms (dimers and trimers) (Figure 3a).

### Example 3:

This Example describes the determination of Anti-F antibody titres.

Nunc-MaxiSorp plate wells were coated overnight at room temperature with 2.5 ng of immunoaffinity-purified RSV-F protein diluted in 0.05M carbonate-bicarbonate buffer, pH9.6. Wells were blocked for non-specific binding by adding 0.1% BSA in PBS for 30 min. at room temperature, followed by two washes in a washing buffer of 0.1% BSA in PBS + 0.1% Tween 20. Serial two or fourfold dilutions of mouse serum was added to the wells. After one hour incubation at room temperature, plates were washed five times with washing buffer, and horseradish peroxidase (HRP) labelled conjugate was added at the appropriate optimal dilution in washing buffer. The total IgG assay used F(ab')₂ goat anti-mouse IgG (H+L specific)-HRP from Jackson Immuno Research Laboratory Inc., Baltimore, Maryland. Sheep anti-mouse IgG1-antibody HRP from Serotec, Toronto, Ontario was used in the IgG1 assay and goat anti-mouse IgG2a antibody from Caltag Laboratories, San Francisco, California was used in the IgG2a assay. Following one hour incubation at room temperature, the plates were washed five times with washing buffer, and hydrogen peroxide (substrate) in the presence of tetramethylbenzidine was added. The reaction was stopped by adding 2 M sulfuric acid. The colour was read in a Multiscan Titertek plate reader at an optical density (OD) of 450 nm. The titre was taken as the reciprocal of the last dilution at which the OD was approximately double. This OD must be greater that the negative control of the assay at the starting dilution. The pre-immune serum of each animal was used as the negative control.

### Example 4:

This Example describes the immunization of animals by priming with the live attenuated *ts* RSV mutant and boosting with RSV proteins.

Pathogen-free BALB/c mice (approximately 8 weeks old; 17 animals per group) were immunized according to the immunization protocol outlined in Table 2. Animals were bled 4 weeks after the primary inoculation and boosted with the antigens shown in Table 2. Serum samples were also taken 4 weeks after the booster dose. The anti-RSV F responses in the sera of BALB/c mice that were immunized according to the prime/boost protocols outlined in Table 2 are summarized in Figure 4. With the exception of the placebo control animals, all mice had anti-F IgG antibodies in their serum. The sera from animals that were primed with the *ts* mutant and boosted with either the *ts* mutant or the subunit preparation had anti-F IgG1/IgG2a ratios of approximately 1. This is in contrast to the anti-RSV F IgG1/IgG2a ratios obtained in mice that were primed and boosted with the alum-adjuvanted FI-RSV or subunit preparation. In this case, anti-RSV IgG1/IgG2a ratios were approximately 3.5 following primary immunization and approximately 2 following the booster dose. These results indicate that priming animals with the ts mutant results in a balanced anti-RSV F IgG1/IgG2a response following the booster dose and sets the stage for a Th-1 type response.

As shown in Figure 5, the sera of mice that were primed and boosted with the various RSV preparations outlined in Table 2, all had significant levels of RSV-specific neutralizing antibodies. The highest RSV-specific neutralizing antibody titers (15.6 log₂) were observed in animals that were primed with the ts mutant and boosted with the RSV subunit preparation. Thus, priming with the *ts* mutant not only appears to set the stage for a Th-1 type response but also results in the induction of high titres of RSV-specific neutralizing antibodies after boosting with the alum-adjuvanted RSV subunit vaccine.

### Example 5:

This Example describes the generation of RSV-specific cytotoxic T-cells in mice primed with live attenuated *ts* RSV and boosted with RSV proteins.

Spleens from two BALB/c mice from each group that were immunized according to the immunization protocol outlined in Table 2 were removed three weeks after the booster dose. Single cell suspensions were prepared and incubated at 2.5x10⁷ cells in RPMI 1640 plus 10% fetal bovine serum (FBS). Gamma-irradiated (3,000 rads) syngeneic spleen cells were infected with RSV at an M.O.I. of 1 for 2 hours. The cells were washed twice to remove free virus and 2.5x10⁷ virus infected feeder cells were added to the 2.5x10⁷ spleen cells in a final volume of 10 mL of complete medium. CTL activity was tested 5 to 6 days following re-stimulation. On the day of the assay, effector cells were washed twice with fresh medium and viable cell counts were determined by the Trypan blue dye exclusion method. BC cells (2 x 10⁶ cells ), a BALB/c fibroblast cell line, as well as BCH4 cells (2 x 10⁶ cells), a BALB/c fibroblast T cell line persistently infected with RSV, were pulsed with 200 µCi of Sodium ⁵¹chromate (Dupont) for 90 min. The targets were washed three times with medium to remove free ⁵¹chromium. Viable cell counts of the target cells were determined and target cell suspensions were prepared at 2 x 10⁴ cells/mL. Washed responder T-cells (in 100 µL) were incubated with 2 x 10³ target cells (in 100 µL) at varying Effector:Target cell ratios in triplicates in a 96-well V-bottomed tissue-culture plates for 4 hours at 37°C with 6% CO₂. Spontaneous and total release of ⁵¹chromium were determined by incubating target cells with either medium or 2.5% Triton X-100 (RTM) in the absence of responder lymphocytes respectively. Six replicates of each were prepared. After 4 hours, plates were centrifuged at 200 x g for 2 min and 100 µL of supernatant were removed from each well to determine the amount of ⁵¹chromium released. Percentage specific ⁵¹chromium release was calculated as (Experimental Release - Spontaneous Release)/(Total Release - Spontaneous Release) x 100. The Spontaneous Release of ⁵¹chromium in the absence of effector cells was found to be between 10 to 15% in these studies. As shown in Figure 6, CTLs generated from mice that were primed with the ts mutant and boosted with the subunit preparation (□), or primed and boosted with the ts mutant (Δ) lysed BCH4 cells (persistently infected with RSV) at all effector dilutions when compared to the lysis of BC (control) cells. CTL activity was not observed in animals that were primed and boosted with either the alum-adjuvanted RSV subunit vaccine (◇) or FI-RSV (■). Thus, priming animals with the ts mutant and boosting them with either the ts mutant or alum-adjuvanted RSV subunit vaccine induced significant levels of CTL activity.

### SUMMARY OF DISCLOSURE

In summary of this disclosure, the present invention provides compositions for a novel immunization strategy to provide protection against disease caused by RSV which is safe and effective.

**Table 1:**

| **Infectivity titres of mutant and wild type virus in MRC-5 cells (day 14)** | | |
|---|---|---|
| VIRUS | **TCID**_{**50**}**/ML** **33°C** | **TCID**_{**50**}**/ML** **39°C** |
| Line 19 MRC-56-35 | 1.00 X 10⁸ | 3.16 X 10⁵ |
| WRSV MRC | 3.16 X 10⁴ | 1.00 X 10⁴ |

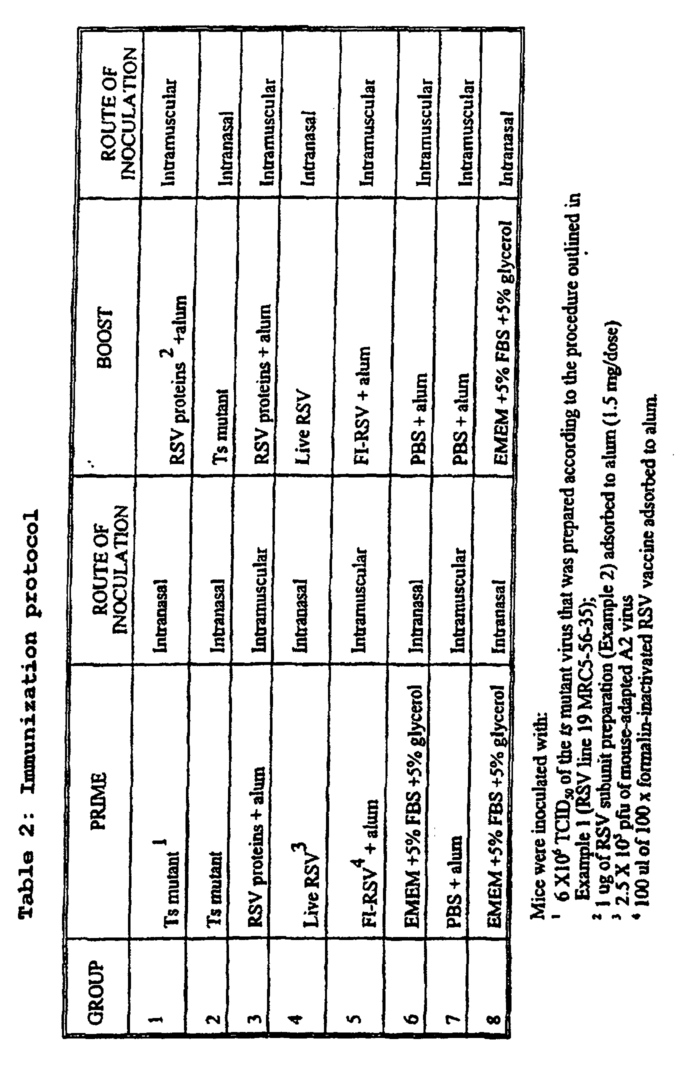

### REFERENCES:

1. Glezen, W.P., Paredes, A. Allison, J.E., Taber, L.H. and Frank, A.L. (1981). J. Pediatr. 98, 708-715.
2. Chanock, R.M., Parrot, R.H., Connors, M., Collins, P.L. and Murphy, B.R. (1992) Pediatrics 90, 137-142.
3. Martin, A. J., Gardiner, P.S. and McQuillin, J. (1978). Lancet ii, 1035-1038.
4. Robbins, A., and Freeman, P. (1988) Sci. Am. 259, 126-133.
5. Glezen, W.P., Taber, L.H., Frank, A.L. and Kasel, J.A. (1986) Am. J. Dis. Child. 140, 143-146.
6. Katz, S.L. New vaccine development establishing priorities. Vol. 1. Washington: National Academic Press. (1985) pp. 397-409.
7. Wertz, G.W., Sullender, W.M. (1992) Biotech. 20, 151-176.
8. Fulginiti, V.A., Eller, J.J., Sieber, O.F., Joyner, i.W., Minamitani, M. and Meiklejohn, G. (1969) Am. J. Epidemiol. 89 (4), 435-448.
9. McIntosh, K. and Chanock, R.M. (1990) in Fields Virology (Fields, B.M., and Knipe, D.M. eds.) pp. 1045-1075, Raven Press, Ltd., New York.
10. Cherrie, A.H., Anderson K, Wertz GW, and Openshaw PJM, 1992, J. Virology, 66: 2102-2110
11. Chin, J., Magoffin, R.L., Shearer, L.A., Schieble, J.H. and Lennette, E.H. (1969) Am. J. Epidemiol. 89 (4), 449-463.
12. Kapikian, A.Z., Mitchell, R.H., Chanock, R.M., Shvedoff, R.A. and Stewart, C.E. (1 9 6 9) Am. J. Epidemiol. 89 (4), 405-421.
13. Connors, M., Collins, P.L., Firestone, C.Y., Sotnikov, A.V., Waitze, A., Davis, A.R., Hung, P.P., Chanock, R.M., Murphy, b. (1992) Vaccine, 10, 475-484.
14. Walsh, E.E., Hall, C.B., Briselli, M., Brandiss, M.W. and Schlesinger, J.J. (1987) J. Infect. Dis. 155 (6), 1198-1204.
15. Connors M, Kulkarni, CY, Firestone KL, Holmes KL, Morse III HC, Sotnikov AV, and Murphy BR, (1992). J. Virol. 66: 7444-7451.
16. Connors M, Giese NA, Kulkarni AB, Firestone CY, Morse III HC, and Murphy BR, (1994) J. Viroi. 68: 5321-5325.
17. Waris, ME, Tsou C., Erdman DD, Zaki SR and Anderson., (1996), J. Virol. 70: 2852-2860.
18. Fulginiti, V.A., Eller, J.J., Sieber, O.F., Joyner, J.W., Minamitani, M. and Meiklejohn, G. (1969) Am. J. Epidemiol. 89 (4), 435-448.
19. Chin, J., Magoffin, R.L., Shearer, L.A., Schieble, J.H. and Lennette, E.H. (1969) Am. J. Epidemiol. 89 (4), 449-463.
20. Jensen, K.E., Peeler, B.E. and Dulworth, W.G. (1962) J. Immunol. 89, 216-226.
21. Murphy, B.R., Prince, G.A., Collins, P.L., Van Wyke Coelingh, K., Olmsted, R.A., Spriggs, M.K., Parrott, R.H., Kim, H.-Y., Brandt, C.D. and Chanock, R.M. (1988) Vir. Res. 11, 1-15.
22. Hall, S.L., Sarris, C.M., Tierney, E.L., London, W.T., and Murphy, B.R. (1993) J. Infect. Dis. 167, 958-962.
23. Belshe, R.B., Karron, R.A., Newman, F.K., Anderson, E.L., Nugent, S.L., Steinhoff, M., Clements, M.L., Wilson, M.H., Hall, S.L., Tierney, E.L. and Murphy, B.R. (1992) J. Clin. Microbiol. 30 (8), 2064-2070.
24. Hall, S.L., Stokes, A., Tierney, E.L., London, W.T., Belshe, R.B., Newman, F.C. and Murphy, B.R. (1992) Vir. Res. 22, 173-184.
25. Van Wyke Coelingh, K.L., Winter, C.C., Tierney, E.L., London, W.T. and Murphy, B.R. (1988) J. Infect. Dis. 157 (4), 655-662.
26. Ray, R., Novak, M., Duncan, J.D., Matsuoka, Y. and Compans, R.W. (1993) J. Infect. Dis. 167, 752-755.
27. Ray, R., Brown, V.E. and Compans, R.W. (1985) J. Infect. Dis. 152 (6), 1219-1230.
28. Ray, R. and Compans, R.W. (1987) J. Gen. Virol. 68, 409-418.
29. Ray, R., Glaze, B.J., Moldoveanu, Z. and Compans, R.W. (1988) J. Infect. Dis. 157 (4), 648-654.
30. Ray, R., Matsuoka, Y., Burnett, T.L., Glaze, B.J. and Compans, R.W. (1990) J. Infect. Dis. 162, 746-749.
31. Ray, R., Glaze, B.J. and Compans, R.W. (1988) J. Virol. 62 (3), 783-787.
32. Ewasyshyn, M., Caplan, B., Bonneau A.-M., Scollard, N., Graham, S., Usman, S. and Klein, M. (1992) Vaccine 10 (6), 412-420.
33. Ambrose, M.W., Wyde, P.R., Ewasyshyn, M., Bonneau, A.-M., Caplan, B., Meyer, H.L. and Klein, M. (1991) Vaccine 9, 505-511.
34. Kasel, J.A., Frank, A.L., Keitel, W.H., Taber, L.H., Glezen W.P. J. Virol. 1984; 52:828-32.
35. Lehman, D.J., Roof, L.L., Brideau, R.J., Aeed, P.A., Thomsen, D.R., Elhammer, A.P., Wathen, M.W. and Homa, F.L. (1993) J. Gen. Virol. 74, 459-469.
36. Brideau, R.J., Oien, N.L., Lehman, D.J., Homa, F.L. and Wathen, M.W. (1993) J. Gen. Virol. 74, 471-477.
37. Ebata, S.N., Prevec, L., Graham, F.L. and Dimock, K. (1992) Vir. Res. 24, 21-33.
38. Hall, S.L., Murphy, B.R. and Van Wyke Coelingh, K.L. (1991) Vaccine 9, 659-667.

## Claims

1. Use of an attenuated strain of a respiratory syncytial virus (RSV) and at least one purified respiratory syncytial virus protein or immunogenic fragment thereof in the manufacture of a medicament for achieving a respiratory syncytial virus specific protective immune response in a host by initially administering to the host an immunoeffective amount of an attenuated respiratory syncytial virus strain, and subsequently administering to the host an immunoeffective amount of at least one purified respiratory syncytial virus protein or immunogenic fragment thereof.

2. The use claimed in claim 1, wherein said immune response in the host includes the production of virus specific neutralizing antibodies and/or virus specific cytotoxic T-cell responses.

3. The use claimed in claim 1 or 2, wherein said attenuated strain of RSV is a temperature sensitive mutant of RSV.

4. The use claimed in any one of claims 1 to 3, wherein said at least one purified RSV protein or immunogenic fragment thereof is selected from the group consisting of the fusion (F), attachment (G) and matrix (M) proteins of RSV or immunogenic fragments thereof.

5. The use claimed in any one of claims 1 to 4, wherein the at least one purified protein or immunogenic fragment thereof is administered with an adjuvant or immunomodulator.

6. The use claimed in claim 5, wherein the adjuvant is alum.

7. A product comprising an immunoeffective amount of (a) an attenuated strain of a respiratory syncytial virus (RSV) and (b) at least one purified respiratory syncytial virus protein or immunogenic fragment thereof as a combined preparation for sequential use as a vaccine against disease caused by respiratory syncytial virus.

8. A product claimed in claim 7, wherein said at least one purified RSV protein or immunogenic fragment thereof is selected from the group consisting of the fusion (F), attachment (G) and matrix (M) proteins of RSV or immunogenic fragments thereof.

9. A product claimed in claim 7 or claim 8, wherein the at least one purified respiratory syncytial virus protein or immunogenic fragment thereof further includes an adjuvant or immunomodulator.

10. A product claimed in claim 9, wherein the adjuvant is alum.

## Patentansprüche

1. Verwendung eines abgeschwächten Stammes eines Respiratory Syncytial Virus (RSV) und wenigstens eines gereinigten Proteins des Respiratory Syncytial Virus oder eines immunogenen Fragmentes von diesem bei der Herstellung eines Medikaments zur Erzielung einer schützenden für das Respiratory Syncytial Virus spezifischen Immunantwort in einem Wirt durch das anfängliche Verabreichen an den Wirt einer immuneffektiven Menge eines abgeschwächten Stammes des Respiratory Syncytial Virus und das anschließende Verabreichen an den Wirt einer immuneffektiven Menge wenigstens eines gereinigten Proteins des Respiratory Syncytial Virus oder eines immunogenen Fragmentes von diesem.

2. Die Verwendung, die in Anspruch 1 beansprucht wird, wobei die Immunantwort in dem Wirt die Erzeugung virusspezifischer neutralisierender Antikörper und/oder virusspezifischer cytotoxischer T-Zellantworten einschließt.

3. Die Verwendung, die in Anspruch 1 oder 2 beansprucht wird, wobei der abgeschwächte Stamm von RSV eine temperaturempfindliche Mutante von RSV ist.

4. Die Verwendung, die in irgendeinem der Ansprüche 1 bis 3 beansprucht wird, wobei das wenigstens eine gereinigte RSV-Protein oder das immunogene Fragment von diesem ausgewählt ist aus der Gruppe, bestehend aus den Fusions (F)-, Anheftungs (G)- und Matrix (M)-Proteinen von RSV oder immunogenen Fragmenten von diesen.

5. Die Verwendung, die in irgendeinem der Ansprüche 1 bis 4 beansprucht wird, wobei das wenigstens eine gereinigte Protein oder das immunogene Fragment von diesem mit einem Adjuvans oder Immunmodulator verabreicht wird.

6. Die Verwendung, die in Anspruch 5 beansprucht wurde, bei welcher das Adjuvans Alaun ist.

7. Ein Produkt, welches eine immuneffektive Menge von (a) einem abgeschwächten Stamm eines Respiratory Syncytial Virus (RSV) und (b) wenigstens ein gereinigtes Protein des Respiratory Syncytial Virus oder ein immunogenes Fragment von diesem umfasst, als kombiniertes Präparat zur aufeinanderfolgenden Anwendung als ein Impfstoff gegen eine Krankheit, welche durch das Respiratory Syncytial Virus verursacht wird.

8. Ein Produkt, welches in Anspruch 7 beansprucht wird, wobei das wenigstens eine gereinigte RSV-Protein oder das immunogene Fragment von diesem ausgewählt ist aus der Gruppe, bestehend aus den Fusions (F)-, Anheftungs (G)- und Matrix (M)-Proteinen von RSV oder immunogenen Fragmenten von diesen.

9. Ein Produkt, welches in Anspruch 7 oder 8 beansprucht wird, wobei das wenigstens eine gereinigte Protein des Respiratory Syncytial Virus oder das immunogene Fragment von diesem weiterhin ein Adjuvans oder einen Immunmodulator einschließt.

10. Ein Produkt, welches in Anspruch 9 beansprucht wird, wobei das Adjuvans Alaun ist.

## Revendications

1. Utilisation d'une souche atténuée d'un virus syncytial respiratoire (RSV) et d'au moins une protéine de virus syncytial respiratoire purifiée ou d'un fragment immunogène de celle-ci dans la production d'un médicament pour obtenir une réponse immunitaire protectrice spécifique de virus syncytial respiratoire chez un hôte par administration initiale à l'hôte d'une quantité immunoefficace d'une souche de virus syncytial respiratoire atténuée, puis administration à l'hôte d'une quantité immunoefficace d'au moins une protéine de virus syncytial respiratoire purifiée ou d'un fragment immunogène de celle-ci.

2. Utilisation selon la revendication 1 où ladite réponse immunitaire chez l'hôte inclut la production d'anticorps neutralisants spécifiques de virus et/ou de réponses à cellules T cytotoxiques spécifiques de virus.

3. Utilisation selon la revendication 1 ou 2 où ladite souche atténuée de RSV est un mutant de RSV sensible à la température.

4. Utilisation selon l'une quelconque des revendications 1 à 3 où ladite ou lesdites protéines de RSV purifiées ou le ou les fragments immunogènes de celles-ci est ou sont choisis dans le groupe consistant en les protéines de fusion (F), de fixation (G) et de matrice (M) de RSV et leurs fragments immunogènes.

5. Utilisation selon l'une quelconque des revendications 1 à 4 où ladite ou lesdites protéines purifiées ou le ou les fragments immunogènes de celles-ci sont administrés avec un adjuvant ou immunomodulateur.

6. Utilisation selon la revendication 5 où l'adjuvant est l'alun.

7. Produit comprenant une quantité immunoefficace de (a) une souche atténuée d'un virus syncytial respiratoire (RSV) et (b) au moins une protéine de virus syncytial respiratoire purifiée ou un fragment immunogène de celle-ci sous forme d'une préparation combinée destinée à être utilisée successivement comme vaccin contre une maladie due à un virus syncytial respiratoire.

8. Produit selon la revendication 7 où ladite ou lesdites protéines de RSV purifiées ou le ou les fragments immunogènes de celles-ci sont choisis dans le groupe consistant en les protéines de fusion (F), de fixation (G) et de matrice (M) de RSV et leurs fragments immunogènes.

9. Produit selon la revendication 7 ou la revendication 8 où la ou les protéines de virus syncytial respiratoire purifiées ou le ou les fragments immunogènes de celles-ci comprennent en outre un adjuvant ou immunomodulateur.

10. Produit selon la revendication 9 où l'adjuvant est l'alun.
